# EUROPEAN PATENT APPLICATION

(11) **EP 2 679 285 A2**
(43) Date of publication of application: **01.01.2014**
(21) Application number: 13173960.9
(22) Date of filing: 27.06.2013
(51) Int. Cl.: A62B 21/00

(54) **Chemical oxygen generator with quick startup properties**

(30) Priority: 28.06.2012 US 201261665486 P
(71) Applicant: INTERTECHNIQUE, 78373 Plaisir Cedex (FR)
(72) Inventor: Rittner, Wolfgang, 23623 Ahrensbök (DE); Meckes, Rüdiger, 23919 Berkenthin (DE); Boomgaarden, Günter, 23684 Scharbeutz (DE); Hollm, Marco, 25548 Rosdorf (DE)
(74) Representative: Eisenführ Speiser

(57) **Abstract**

The invention relates to a chemical oxygen generator for an emergency oxygen supply device in an aircraft, said generator comprising a housing,a solid chemical substance comprising oxygen in chemically bound form arranged inside said housing and extending along a longitudinal axis of said housing, a starter unit positioned adjacent to said chemical substance, said starter unit being adapted to provide energy to said chemical substance for initiating a chemical reaction of said solid chemical substance producing gaseous oxygen, an outflow port in said housing being in fluid communication with said chemical substance. According to the invention said solid chemical substance extends from a starter region adjacent to the starter unit to an end region, wherein the contour length to surface ratio of a cross section of said chemical substance in said starter region is larger than the contour length to surface ratio of a cross section of said chemical substance in the end region.

## Description

The invention relates to a chemical oxygen generator for an emergency oxygen supply device in an aircraft, said generator comprising a housing,a solid chemical substance comprising oxygen in chemically bound form arranged inside said housing and extending along a longitudinal axis of said housing, a starter unit positioned adjacent to said chemical substance, said starter unit being adapted to provide energy to said chemical substance for initiating a chemical reaction of said solid chemical substance producing gaseous oxygen, an outflow port in said housing being in fluid communication with said chemical substance.

Such chemical oxygen generators are used to provide oxygen to passengers of an aircraft in an emergency situation like a decompression situation. The chemical generators are usually stored in a ceiling compartment above the passenger. In case of an emergency situation the chemical oxygen generator is activated by starter unit which is coupled to a control unit detecting such emergency situation. The starter unit ignites the chemical substance like sodium chlorate inside the chemical oxygen generator thus starting the chemical reaction of the sodium chlorate to gaseous oxygen and remainders.

A problem associated with the use of such chemical oxygen generators is the startup period. An emergency situation often requires immediate supply of oxygen to the passengers since a rapid decompression may hinder the passenger from further breathing cabin air with sufficient oxygen content. The passenger may thus wish to immediately activate the chemical oxygen generator by pulling the masked towards himself and thus effecting the starting ignition. However, the chemical reaction in the chemical oxygen generator hereafter will be started and this startup period is characterized by a slow start of the chemical reaction producing only little oxygen in the initial phase.

It is generally known to improve the startup by an improved ignition process or an accelerated chemical reaction by specific ignitions of the chemical substance. Whereas this may improve the startup supply of oxygen a general draw back of such measures is the reduced supply time due to the enhanced chemical reaction.

Usually, a civil aircraft flying in high altitude and experiencing a decompression situation will immediately conduct a descent to a lower altitude and then travel at this lower altitude to the next airport. Following such altitude profile in the emergency situation immediate supply of large amounts of oxygen is required right after the emergency situation has occurred in the high altitude flight level. This supply shall be maintained until the aircraft has reached the lower altitude or may even be decreased during descent of the aircraft. When having reached the low altitude the oxygen supply may be reduced to a lower amount to allow a long supply time of the oxygen during flight. It is known to provide control units controlling a control valve unit to adapt the amount of oxygen delivered to the passenger to such an altitude profile or the altitude flight level. However, such control valves have the significant draw back of inducing high pressure in the chemical oxygen generator which vice versa has an influence on the chemical reaction. In particular, a high pressure in the chemical oxygen generator may decrease the chemical reaction to an inacceptable chemical reaction rate and may in particular be critical if the terrestrial ground profile requires to return to a higher flight level during traveling to the next airport, e.g. due to a mountain range to be passed by the aircraft.

It is an object of the invention to provide an emergency oxygen device which better fulfills the requirements of oxygen supply in an emergency situation than prior art systems. It is a particular object of the invention to provide an emergency oxygen device which better addresses the needs of the passenger for oxygen in the distinct flight episodes after an emergency situation.

According to the invention this object is achieved by a chemical oxygen generator as described in the introductory portion before hand, wherein said solid chemical substance extends from a starter region adjacent to the starter unit to an end region, wherein the contour length to surface ratio of a cross section of said chemical substance in said starter region is larger than the contour length to surface ratio of a cross section of said chemical substance in the end region.

According to the invention, an important factor influencing the speed of the chemical reaction is specifically selected by the design of the solid chemical substance inside the housing of the chemical oxygen generator. The invention is based on the inventor recognizing that the chemical substance inside the housing will have an immediate startup of the chemical reaction and a high delivery rate of gaseous oxygen if the chemical substance has a large surface in the region adjacent to the starter unit and preferably a small volume. Generally, both these geometrical properties will accelerate the chemical reaction as a single influencing factor. Thus, when providing a high surface to volume ratio of the chemical substance close to the starter unit an immediate start of the chemical reaction can be reached.

A further aspect of the invention is to provide a different surface to volume ratio of the solid chemical substance. In use, the solid chemical substance will react from the beginning at the starter unit to an end which usually is opposed to said starter unit. The travel path between this beginning at the starter unit and the end may be defined as a chemical reaction path. According to the invention, the surface to volume ratio of the solid chemical substance changes along this chemical reaction path from a high surface to volume ratio to a lower surface to volume ratio. The geometrical options to reach such changing of the surface to volume ratio are multiple and may lie in a change of the geometry of an outer surface of the solid chemical substance, a change of the geometry of an inner surface delimiting an opening inside the solid chemical substance or both. The change in geometry may be a change in dimension or a change in a design or both. By this specific measure according to the invention an immediate start-up of the chemical reaction will be followed by a reduction of the speed of the chemical reaction along the chemical reaction path and thus an elongation of the supply time of oxygen at a lower delivery rate in the time period after start-up.

According to the invention the surface to volume ratio is defined to be a contour lenght to surface ratio of a cross section of said solid chemical substance. This is to be understood as a ratio of the contour or outline length of the cross-section representing the surface and the surface or area of the cross-section representing the volume. It is to be understood that the contour may be defined to be an inner contour of an opening inside the solid chemical substance and extending along the length of the chemical substance or the contour may be defined as an outline of the solid chemical substance or an addition of both. Further, it is to be understood that the surface shall be defined to be the area between such outline and such inner contour if an opening is present, namely that area which is representing the presence of the solid chemical substance in such cross-section.

According to a first preferred embodiment said solid chemical substance is formed with an inside opening, said opening forming a part of the surface of said solid chemical substance, wherein the cross section of said opening has a first contour in the starter region and a second contour in the end region, said first contour being longer than said second contour. This particular embodiment comprises an inside opening extending along the length of the solid chemical substance and usually serving to direct gaseous oxygen produced in the chemical reaction to an outflow port. A main advantage of such design is a preheating effect of the solid chemical substance downstream of the position of the chemical reaction which improves the progress of the chemical reaction throughout the whole solid chemical substance. In such embodiment, the different contour length to surface ratio, i.e. the different surface to volume ratio in the different regions of the solid chemical substance can effectively be achieved by changing the design or dimension of such opening. In one example, the opening may be conical and may change from a large diameter in the starter region to a smaller diameter in the end region. In another, even more effective example the cross-section of the opening in the starter region may have a different geometry in the starter region than in the end region by maintaining the surface of the opening in a cross-section.

It is further preferred that the cross-section of the opening in the starter region is selected from a noncircular cross-section, a star-like cross-section a polygonal cross-section. By selecting a cross-section having any of these designs which are different from a circular cross-sectional design of the opening an improved contour to surface ratio of a cross-section will effectively be achieved. It is to be understood that these different design options for the cross-section in the starter region may in the same way apply to the outline of the solid chemical substance in the starter region and it may in particular be preferred to design an opening and the outline of the solid chemical substance in the starter region according to any of these particular design options.

According to another preferred embodiment the cross-section of the opening in the end region has a circular cross-section. It is to be understood that a circular design of the cross-section, either being directed to an opening or to an outline or both of the cross-section will be an optimum for minimizing the contour length to surface ratio or surface to volume ratio of the solid chemical substance in this region. In particular, the solid chemical substance may have a ring-like shape in the end region.

It is further preferred that the cross-section of the opening has a first geometry in the starter region and a second geometry, which is different from said first geometry in the end region wherein said cross-section changes steady, in particular, uniformly continuously from said starter region to said end region. Generally, it is to be understood that the cross-section of an opening extending along the length of the solid chemical substance and along the reaction travel path or the outline along said direction of the length of the solid chemical substance may be defined by two distinct regions having a constant contour length to surface ratio each and thus having an immediate change of this ratio, e.g. in order to adapt the delivery rate of oxygen per time unit to the flight profile in an emergency situation. In the same way, three distinct regions having three distinct contour length to surface ratios may be provided. In this particular embodiment, however, a steady change of the cross-section from the starter region to the end region may be provided effecting a rather steady change of the delivery rate of oxygen over the time of the chemical reaction.

This change may in particular be uniformly continuous at least in a section of the length of the solid chemical substance to provide a continuous reduction of the delivery rate at a certain time period during the emergency flight profile.

According to a further aspect of the invention a chemical oxygen generator is provided according to the characteristics described in the introductory portion wherein said starter unit is positioned to initiate said chemical reaction in a starter region of said solid chemical substance, said starter region being positioned between said first end and said second end.

According to this aspect of the invention the starter unit is positioned to initiate the chemical reaction at a position of the solid chemical substance which is in a distance to both ends of the solid chemical substance which extends from one end to another end. Thus, in contrast to prior art systems wherein the chemical reaction is initiated at one end point and then travels along a single chemical reaction path to another end of the solid chemical substance, in this embodiment the chemical reaction is started at one point and then travels along two chemical reaction paths to both ends of the solid chemical substance. By this, the chemical reaction takes place simultaneously at two different locations within the solid chemical substance and thus the delivery rate is significantly improved immediately after start-up of the chemical reaction. The chemical reaction then progresses along the longitudinal axis of the solid chemical substance in a first direction to the first end and in second direction to a second end wherein the first and second direction are opposite to each other.

The starter unit may be positioned in a particular position between the first and the second end such that a first volume is present between said position and the first end of the solid chemical substance and a second volume is present between said position and the second end of the solid chemical substance. Generally, the position of the starter unit is not to be interpreted to define the position of a component which fulfills the starting function and thus should not be understodd to delimit the invention to an oxygen generator having such a component in in such a particular position. Instead, the term starter unit shall be understood to define the particular element which initiates the start, like e.g. the spark igniting the chemical substance. The starter unit may be positioned by providing a starter component at the particular position, e.g. by radially extending into the chemical oxygen generator between said first and second end. The starter unit may be positioned by providing a starter component mounted at the first or the second end and extending axially to a to a position between said first and second end.

It is particularly preferred that wherein said starter unit is positioned at a start position of the solid chemical substance, a first volume of the solid chemical substance being arranged in the region between the start position and the first end and a second volume of the solid chemical substance being arranged in the region between the start position and the second end, wherein the first volume is preferably smaller than the second volume. According to this embodiment, the chemical reaction of the solid chemical substance takes place in two positions along two separate chemical reaction paths. The volumes of the solid chemical substance which is reacting along these two distinct chemical reaction paths is different thus effecting the chemical reaction in the first volume to be limited to a reaction time which is shorter than the chemical reaction in the second volume. Thus, the supply rate of oxygen per time unit will be initially high after start-up of the chemical reaction due to the reaction taking place in both the first and the second volume but will only be high as long as the reaction takes place in both volumes. After the first volume of the solid chemical substance is exhausted and has reacted completely the chemical reaction will only take place in the second volume in further progress. By this, a specific profile of the delivery rate which is particularly adapted to the flight altitude profile of an aircraft in an emergency situation may be configured in that after exhaustion of the first volume the second volume will deliver oxygen at a low rate for a long supply time corresponding to the low altitude flight level and the possible long flight time to the next airport.

According to a further preferred embodiment said starter unit is positioned at a start position of the solid chemical substance, a first volume of the solid chemical substance being arranged in the region between the start position and the first end and a second volume of the solid chemical substance being arranged in the region between the start position and the second end, further comprising a first flow path for oxygen generated in the first volume, said first flow path being directed from the first volume to the outflow port and a second flow path for oxygen generated in the second volume said second flow path being directed from the second volume to the outflow port, wherein a first flow path section of said first flow path adjacent to said first volume has a direction from the start position to the first end and a second flow path section of said second flow path adjacent to said second volume has a direction from said second end to said start position. This particular embodiment is further improved with regard to the supply rate of oxygen out of the first volume and the second volume. This embodiment is based on the inventor recognizing that the chemical reaction in a volume is accelerated if the gaseous oxygen flows from the point of reaction in this volume through the rest of the volume which not yet has undergone the chemical reaction. By this, the rest of the volume is preheated by the gaseous oxygen which improves the speed of the chemical reaction in this preheated volume. In contrast, if such preheating is prevented by directing the gaseous oxygen in opposite direction to the reaction travel path the chemical reaction is decelerated resulting in a long delivery time of oxygen at low oxygen delivery rate. According to this embodiment the flow path of the gaseous oxygen is directed in the direction of the chemical reaction path in the first volume and opposite to the direction of the chemical reaction path in the second volume. Thus, the chemical reaction is accelerated by a preheating effect in the first volume and is decelerated by a non-preheating effect in the second volume. This further enhances the quick start-up and high delivery rate of oxygen in an initial time period right after start-up of the chemical oxygen generator and improves the length of the delivery rate in a subsequent time period out of the second volume.

It is in particular preferred that said solid chemical substance has an opening extending from said first end to said second end and wherein the first flow path section and the second flow path section is inside said opening. By directing the gaseous oxygen through such an opening the effect of preheating or non-preheating can be enhanced effectively. The solid chemical substance may have such an opneing over its entire lengthj or only in a section whichj extends along a part opf said length. The solid chemical substance may be completely filled, i.e. without such opening, in an alternative em,bodiment hereto, too. In such case, the cross section of the solid chemical substance is entirely filled over the entire length of the solid chemcial substance.

According to a further aspect of the invention a chemical oxygen generator for an emergency oxygen supply device in an aircraft is provided said generator comprising a plurality of housings, each housing comprising a solid chemical substance comprising oxygen in a chemically bound form arranged inside said housing and extending along a longitudinal axis of said housing from a first end to a second end, each housing comprising a starter unit mounted to said housing and positioned adjacent to said chemical substance, said starter unit being adapted to provide energy to said chemical substance for initiating a chemical reaction of said solid chemical substance producing gaseous oxygen, each housing comprising an outflow port in said housing being in fluid communication with said chemical substance, wherein said outflow ports are connected to a manifold to connect said outflow ports, characterized by a control unit coupled to said starter units, wherein said control unit is adapted to simultaneously activate at least two starter units. According to this aspect, the oxygen is supplied to one single oxygen mask out of two or more chemical oxygen generators simultaneously. According to this embodiment it is not one single large chemical oxygen generator which is used to supply oxygen to one single passenger or a group of passengers but instead a plurality of small oxygen generator like e.g. two, three or four chemical oxygen generators is used for such supply to a single passenger or a group of passengers. It is to be understood that the single passenger or each passenger out of the group of passengers is supplied out of all simultaneously ignited chemical oxygen generators in this embodiment simultaneously. This aspect of the invention is based on the inventor recognizing that start-up of a plurality of small chemical oxygen generators will result in a initially higher delivery rate of oxygen when compared to start-up of one single large chemical oxygen generator having the same amount of solid chemical substance like the four small chemical oxygen generators in sum. Thus, the initial delivery rate can be improved significantly without providing a larger amount of solid chemical substance here.

It is particularly preferred that said control unit is adapted to control the starter units according to any of the following schemes: simultaneously activating all starter units, simultaneously activating a first subset of the plurality of starter units and subsequently activating the starter units of a second subset one after the other, wherein the first subset comprises at least two starter units simultaneously activating a first subset of the plurality of starter units and subsequently simultaneously activating a second subset of the plurality of starter units and subsequently activating the starter units of a third subset one after the other, wherein the first subset and the second subset each comprises at least two starter units. According to this invention, the advantage of starting the chemical reaction in two or more chemical oxygen generators simultaneously at the beginning of the oxygen supply right after occurrence of the emergency situation it combined with a subsequent start-up of the chemical reaction in further chemical oxygen generators to elongate the delivery time of oxygen out of the emergency oxygen device. It is to be understood that in these subsequent start-ups of the chemical reaction a single or a plurality of oxygen generators may be ignited simultaneously to maintain a certain delivery rate and that the selection of the number of oxygen generators to be ignited may depend on the flight altitude level or the oxygen consumption of the passenger which may be measured by respective sensors and input in a control unit controlling the ignition of the chemical oxygen generators.

Whereas different aspects and embodiments have been described beforehand it is to be understood that the different aspects and characteristics of these embodiments may be combined with each other to reach a design and control mechanism of an emergency oxygen device which is in particular helpful to provide a delivery rate of oxygen adapted to a rapid start-up and a long-term delivery rate of oxygen during the flight after occurrence of an emergency situation.

A further aspect of the invention is A method of providing oxygen to a passenger of an aircraft, wherein gaseous oxygen is produced in a chemical reaction of a solid chemical substance containing oxygen in a chemically bound form and said gaseous oxygen is provided to the passenger via an oxygen mask, **characterized in that** the gaseous oxygen is produced in a start-up time period by a starter region of said chemical substance and in a subsequent time period by an end region of said chemical substance, wherein said starter region has a larger surface-to-volume ration than said end region in particular a larger contour length to surface ratio in a cross-section than said end region.

Still further, an aspect of the invention is A method of providing oxygen to a passenger of an aircraft, wherein gaseous oxygen is produced in a chemical reaction of a solid chemical substance containing oxygen in a chemically bound form and said gaseous oxygen is provided to the passenger via an oxygen mask, **characterized in that** the gaseous oxygen is produced by starting the chemical reaction in a mid portion of said solid chemical substance and said chemical substance reacts starting from said mid portion in a first reaction path extending from the mid portion to a first end of said chemical substance and a second reaction path extending from the mid portion to a second end of said chemical substance, simultaneously.

Referring to this embodiment it is particularly preferred that the gaseous oxygen produced in a first part of the chemical substance extending from the mid portion to the first end is directed from the chemical substance to an outflow port in the direction of the first reaction path, and the gaseous oxygen produced in a second part of the chemical substance extending from the mid portion to the second end is directed from the chemical substance to an outflow port against the direction of the first reaction path.

Finally a further aspect of the invention is A method of providing oxygen to a passenger of an aircraft, wherein gaseous oxygen is produced in a chemical reaction of a solid chemical substance containing oxygen in a chemically bound form and said gaseous oxygen is provided to the passenger via an oxygen mask, **characterized in that** the gaseous oxygen is produced by simultaneously starting at two chemical reactions in a first container comprising a part of the chemical substance and a second container comprising a part of the chemical substance, simultaneously and providing gaseous oxygen out of said two chemical reactions to a single oxygen mask.

Regarding these aspects of the methods according to the invention it is to be understood that the method may in particular be conducted using a chemical oxygen generator as described beforehand and as claimed in the claims. Thus, regarding the advantage and preferred embodiments of these aspects of the method according to the invention reference is made to the foregoing description of the corresponding advantages, functionalities and preferred embodiments of the chemical oxygen generator.

Preferred embodiments of the invention are described in detail below with reference to the figures.
Figure 1 shows a schematical cross-sectional view of a chemical oxygen generator according to preferred embodiment of the invention.
Figure 2 shows a cross-sectional view of the solid chemical substance according to a preferred design with large surface to volume ratio.
Figure 3 shows a cross-sectional view according to figure 2 with a reduced surface to volume ratio.
Figure 4 shows a schematical setup of an embodiment of a chemical oxygen generator unit according to the invention.

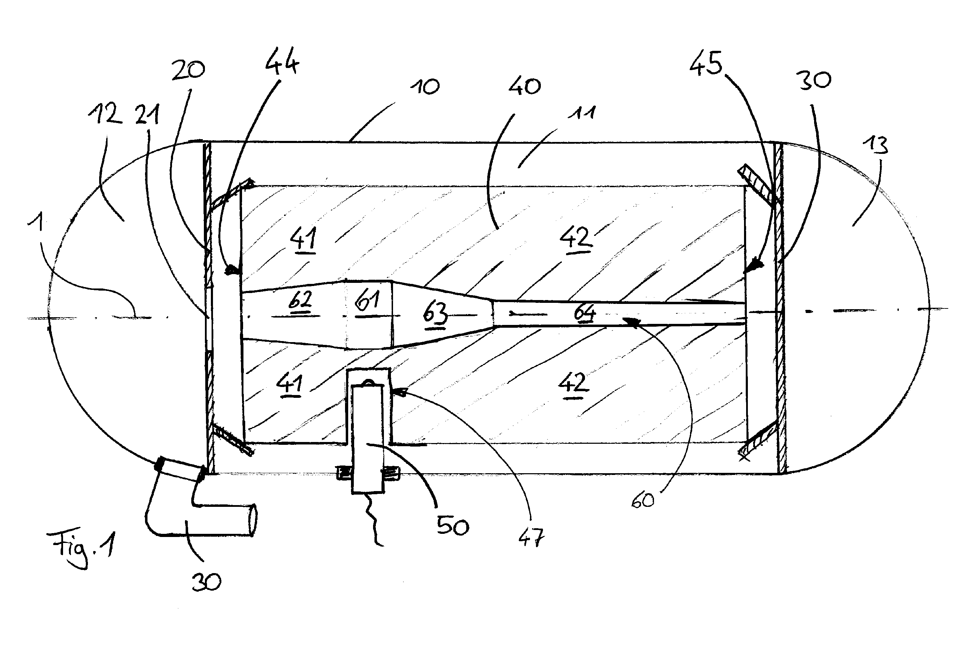

Referring first to figure 1, a chemical oxygen generator comprises a housing 10, which extends in a longitudinal direction along a longitudinal axis 1. The housing 10 is to be understood to be of general cylindrical design with spherical endplates as shown in figure 1.

Inside the housing 10, two holding plates 20, 30 are positioned adjacent to each end of the housing. The first position plate 20 at the first end of the housing comprises a central opening 21 allowing gaseous oxygen to enter out of an inner cylindrical space 11 inside the housing into a half dome shaped space 12 at the first end of the housing.

An outflow port 30 is inserted into the wall of the housing in the half dome shaped space 12. Gaseous oxygen flowing through the opening 21 in the holding plate 20 into the half dome shaped space 12 may exit the housing through the outflow port 30 and can be supplied to an oxygen mask for providing said oxygen to a passenger.

The holding plates 20, 30 fix a solid chemical substance 40 extending along the longitudinal axis 1 from a first end 44 to a second end 45 inside the cylindrical space 11 of the housing. The solid chemical substance 40 is sodium chlorate and contains oxygen in chemically bound form. The outer surface of the solid chemical substance 40 is of cylindrical shape with constant diameter over the entire length of the solid chemical substance.

An opening 60 extending along the entire length in the longitudinal direction of the solid chemical substance is provided along the center line of the solid chemical substance. The opening 60 is divided into four sections. In a starter section 61 which is directly adjacent to a starter unit 50 inserted into a radial bore 47 in the solid chemical substance a cylindrical cross action of the opening is provided. This starter section 61 provides a high surface to volume ratio or, in other words, the cross section of the solid chemical substance in this starter section 61 has a large contour length to surface ratio.

A first end section 62 of the opening extends from said starter section 61 to the first end 44. This first section is surrounded by a first volume 41 of the solid chemical substance. The first section 62 of the opening 60 has a conical shape with a diameter which constantly reduces in the direction from the starter section 61 to the first end 44.

Further, a transition section 63 of the opening is provided extending from the starter section 61 in the opposite direction to the second end 45. This transition section 63 has a conical shape too, reducing its diameter in the direction from the starter section 61 to the second end 45.

Finally, a second end section 64 of the opening 60 is provided. Said second end section 64 has a cylindrical shape and extends from the transition section 63 to the second end 45.

The transition section 63 and the second end section 64 are surrounded by a second volume 42 of the solid chemical substance.

The starter unit 50 is arranged to ignite and start the chemical reaction in the solid chemical substance surrounding the starter section 61. Due to the high surface to volume ratio in this starter section, the chemical reaction will develop rapidly and an immediate high delivery rate of oxygen will be provided right after ignition by the starter unit 50.

The chemical reaction will then run along two distinct reaction parts, namely towards the first end 44 through the first end section 62 and towards the second end 45 through the transition section 63 and the second end section 64.

Generally, two effects will be observed during this chemical reaction.

First, the chemical reaction along the reaction paths through the first end section and the transition section will be decelerated in course of progress of the chemical reaction due to the reduction of the surface to volume ratio or, in other words, the contour length to surface ratio of the cross-section when progressing towards the first end and the second end, respectively. This is a desired effect to reduce the delivery rate of oxygen per time unit after the initial quick start-up and to preserve solid chemical substance for a long-time delivery of oxygen after the initial start-up time period.

Second, due to the second holding plate being closed any gaseous oxygen which is produced in the chemical reaction has to pass through the opening 21 in the first holding plate 20. Thus, the flow path of the gaseous oxygen is in opposite direction to the direction of the chemical reaction in the transition section and the second end section effecting a further deceleration of the speed of the chemical reactio in these two sections. In contrast, the flow path of the gaseous oxygen is in the same direction like the travel path of the chemical reaction in the first end section effecting a preheating of the solid chemical substance and thus an acceleration of the chemical reaction in this section. As a consequence of this preheating and non-preheating the first volume will react quickly and produce a high delivery rate of oxygen whereas the second volume 42 will react slowly and produce a long delivery time of oxygen. This perfectly fits to the flight altitude profile of an aircraft after a decompression situation has occurred in high altitude.

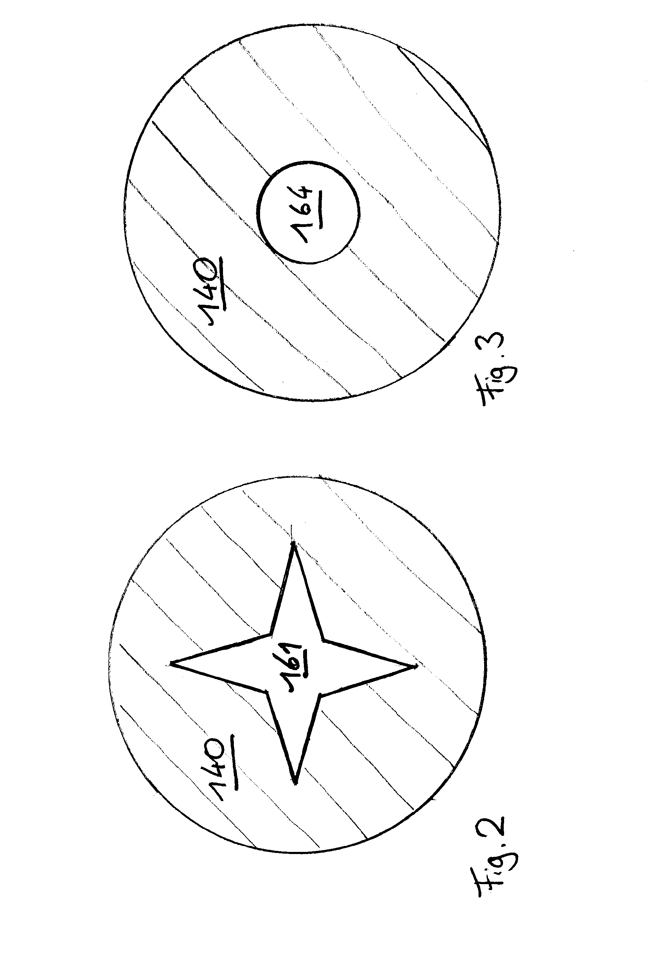

Referring now to figure 2 a preferred cross-sectional design is depicted which could be employed in the starter section of a chemical oxygen generator. As can be seen, a star-shaped cross-sectional opening 161 is provided inside a solid chemical substance 140 wherein the length of the contour of said star-shaped opening is very large without significantly reducing the surface filled by the solid chemical substance of the cross-section. Thus, a high ratio of the contour length versus the surface or, respectively of the surface versus the volume can be achieved this cross-sectional design.

Figure 3 depicts a preferred design of a cross-section of a solid chemical substance which preferably is employed in the second end section, the first end section or the transition section of a solid chemical substance as shown in figure 1. In this design the cross-section includes a cylindrical shaped opening 164 thus effecting a small contour length of this opening and a small ratio of the contour length versus the surface 140 of the cross-section filled by the solid chemical substance.

It is to be understood that various different designs fulfilling the requirements of a large contour length or a small contour length in relation to the surface filled by the solid chemical substance in the cross-section may be employed and used according to the invention.

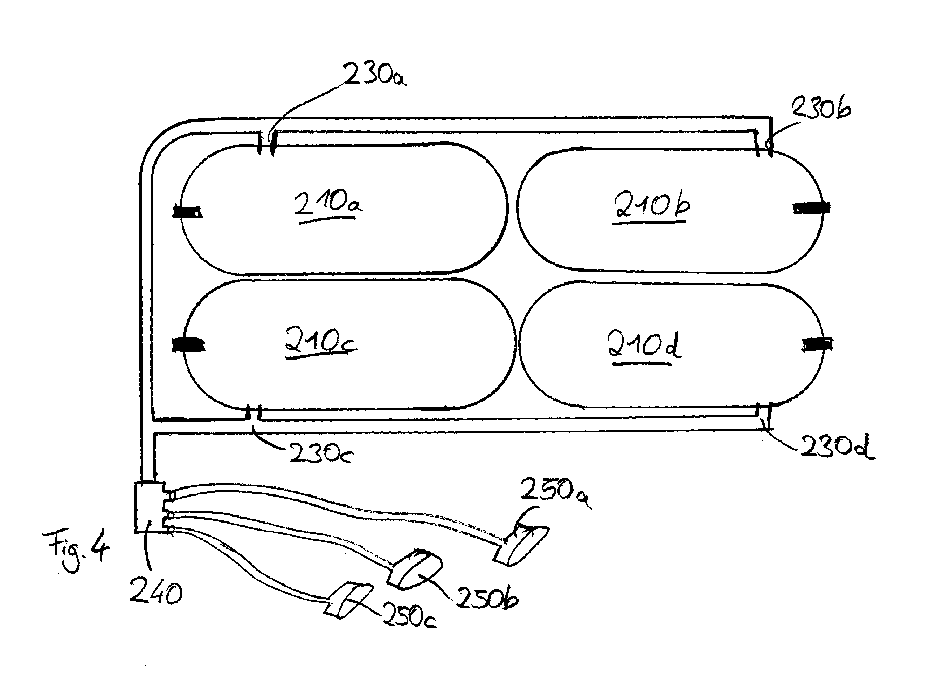

Referring now to figure 4, a set-up of four chemical oxygen generators 210 a, b, c, d is shown. The outflow ports 230 a, b, c, d of these four chemical oxygen generators are connected via oxygen flow lines to a central manifold 240. Said central manifold provides the oxygen flowing out of the oxygen generators 250 a-e to three oxygen masks 250 a-c wherein it is to be understood that each of the oxygen masks 290 a-c is supplied from all four chemical oxygen generators 210 a-d.

According to this embodiment, a control unit (not shown) is provided which simultaneously activates the chemical oxygen generators 210 a, b and c. In this initial start-up the chemical reaction inside these three chemical oxygen generators will quickly develop and provide a high initial delivery rate of oxygen for a start-up time. The control unit is further adapted to ignite the fourth chemical oxygen generator 210 d after a predetermined time to supply oxygen at a low delivery rate for elongating the total delivery rate of oxygen after an emergency situation has occurred.

## Claims

1. Chemical oxygen generator for an emergency oxygen supply device in an aircraft, said generator comprising:
- a housing
- a solid chemical substance comprising oxygen in chemically bound form arranged inside said housing and extending along a longitudinal axis of said housing,
- a starter unit positioned adjacent to said chemical substance, said starter unit being adapted to provide energy to said chemical substance for initiating a chemical reaction of said solid chemical substance producing gaseous oxygen,
- an outflow port in said housing being in fluid communication with said chemical substance,
**characterized in that** said solid chemical substance extends from a starter region adjacent to the starter unit to an end region,
wherein the contour length to surface ratio of a cross section of said chemical substance in said starter region is larger than the contour length to surface ratio of a cross section of said chemical substance in the end region.

2. Generator according to claim 1,
wherein said solid chemical substance is formed with an inside opening, said opening forming a part of the surface of said solid chemical substance, wherein the cross section of said opening has a first contour in the starter region and a second contour in the end region, said first contour being longer than said second contour.

3. Generator according to claim 1 or 2,
wherein the cross-section of the opening in the starter region is selected from
- A noncircular cross-section,
- A star-like cross-section,
- A polygonal cross-section.

4. Generator according to any of the preceding claims,
wherein the cross-section of the opening in the end region has a circular cross-section.

5. Generator according to any of the preceding claims,
Wherein the cross-section of the opening has a first geometry in the starter region and a second geometry, which is different from said first geometry in the end region wherein said cross-section changes steady, in particular uniformly continuously from said starter region to said end region.

6. Chemical oxygen generator for an emergency oxygen supply device in an aircraft, said generator comprising:
- a housing,
- a solid chemical substance comprising oxygen in a chemically bound form arranged inside said housing and extending along a longitudinal axis of said housing from a first end to a second end,
- a starter unit positioned adjacent to said chemical substance, said starter unit being adapted to provide energy to said chemical substance for initiating a chemical reaction of said solid chemical substance producing gaseous oxygen,
- an outflow port in said housing being in fluid communication with said chemical substance,
**characterized in that** said starter unit is positioned to initiate said chemical reaction in a starter region of said solid chemical substance, said starter region being positioned between said first end and said second end

7. Generator according to claim 6,
wherein said starter unit is positioned at a start position of the solid chemical substance, a first volume of the solid chemical substance being arranged in the region between the start position and the first end and a second volume of the solid chemical substance being arranged in the region between the start position and the second end,
wherein the first volume is smaller than the second volume.

8. Generator according to claim 6 or 7,
wherein said starter unit is positioned at a start position of the solid chemical substance, a first volume of the solid chemical substance being arranged in the region between the start position and the first end and a second volume of the solid chemical substance being arranged in the region between the start position and the second end,
Further comprising
- a first flow path for oxygen generated in the first volume, said first flow path being directed from the first volume to the outflow port and
- a second flow path for oxygen generated in the second volume said second flow path being directed from the second volume to the outflow port,
wherein a first flow path section of said first flow path adjacent to said first volume has a direction from the start position to the first end and a second flow path section of said second flow path adjacent to said second volume has a direction from said second end to said start position.

9. Generator according to claim 8,
wherein said solid chemical substance has an opening extending from said first end to said second end and wherein the first flow path section and the second flow path section is inside said opening.

10. Chemical oxygen generator for an emergency oxygen supply device in an aircraft, said generator comprising:
- A plurality of housings, each housing comprising a solid chemical substance comprising oxygen in a chemically bound form arranged inside said housing and extending along a longitudinal axis of said housing from a first end to a second end,
- Each housing comprising a starter unit mounted to said housing and positioned adjacent to said chemical substance, said starter unit being adapted to provide energy to said chemical substance for initiating a chemical reaction of said solid chemical substance producing gaseous oxygen,
- Each housing comprising an outflow port in said housing being in fluid communication with said chemical substance, wherein said outflow ports are connected to a manifold to connect said outflow ports, **characterized by** a control unit coupled to said starter units, wherein said control unit is adapted to simultaneously activate at least two starter units.

11. Generator according to claim 10,
wherein said starter unit is adapted to control the starter units according to any of the following schemes:
- simultaneously activating all starter units,
- simultaneously activating a first subset of the plurality of starter units and subsequently activating the starter units of a second subset one after the other, wherein the first subset comprises at least two starter units
- simultaneously activating a first subset of the plurality of starter units and subsequently simultaneously activating a second subset of the plurality of starter units and subsequently activating the starter units of a third subset one after the other, wherein the first subset and the second subset each comprises at least two starter units.

12. Generator according to claim 10 or 11,
wherein the plurality of housings is arranged inside a single emergency oxygen box.

13. Generator according to any of the preceding claims 6-9,
**characterized by** the features of any of the claims 2-5.

14. A method of providing oxygen to a passenger of an aircraft, wherein gaseous oxygen is produced in a chemical reaction of a solid chemical substance containing oxygen in a chemically bound form and said gaseous oxygen is provided to the passenger via an oxygen mask,
**characterized in that** the gaseous oxygen is produced in a start-up time period by a starter region of said chemical substance and in a subsequent time period by an end region of said chemical substance, wherein said starter region has a larger surface-to-volume ration than said end region in particular a larger contour length to surface ratio in a cross-section than said end region.

15. A method of providing oxygen to a passenger of an aircraft, wherein gaseous oxygen is produced in a chemical reaction of a solid chemical substance containing oxygen in a chemically bound form and said gaseous oxygen is provided to the passenger via an oxygen mask,
**Characterized in that** the gaseous oxygen is produced by starting the chemical reaction in a mid portion of said solid chemical substance and said chemical substance reacts starting from said mid portion in a first reaction path extending from the mid portion to a first end of said chemical substance and a second reaction path extending from the mid portion to a second end of said chemical substance, simultaneously.

16. A method according to claim 15, wherein
- the gaseous oxygen produced in a first part of the chemical substance extending from the mid portion to the first end is directed from the chemical substance to an outflow port in the direction of the first reaction path, and
- the gaseous oxygen produced in a second part of the chemical substance extending from the mid portion to the second end is directed from the chemical substance to an outflow port against the direction of the first reaction path.

17. A method of providing oxygen to a passenger of an aircraft, wherein gaseous oxygen is produced in a chemical reaction of a solid chemical substance containing oxygen in a chemically bound form and said gaseous oxygen is provided to the passenger via an oxygen mask,
**characterized in that** the gaseous oxygen is produced by simultaneously starting at two chemical reactions in a first container comprising a part of the chemical substance and a second container comprising a part of the chemical substance, simultaneously and providing gaseous oxygen out of said two chemical reactions to a single oxygen mask.
